# EUROPEAN PATENT APPLICATION

(11) **EP 4 556 490 A1**
(43) Date of publication of application: **21.05.2025**
(21) Application number: 22951274.4
(22) Date of filing: 30.09.2022
(51) Int. Cl.: C07K 16/10, A61P 31/14, G01N 33/569, A61K 39/00

(54) **SARS-COV-2 NEUTRALIZING ANTIBODY**

(30) Priority: 12.07.2022 KR 20220085495
(71) Applicant: Scripps Korea Antibody Institute, Gangwon-do 24341 (KR)
(72) Inventor: LEE, Sungjin, Chuncheon-si Gangwon-do 24363 (KR); KO, Haeli, Chuncheon-si Gangwon-do 24435 (KR); LEE, Deuk ki, Chuncheon-si Gangwon-do 24264 (KR); KIM, Younghyeon, Namyangju-si Gyeonggi-do 12219 (KR); LEE, Hai-Chon, Chuncheon-si Gangwon-do 24303 (KR)
(74) Representative: V.O.
(86) International application number: PCT/KR2022/014794
(87) International publication number: WO 2024/014619

(57) **Abstract**

The present disclosure relates to a neutralizing antibody against SARS-coronavirus 2 (SARS-CoV2) or a variant virus thereof, or an antigen-binding fragment thereof. Since the neutralizing antibody of the present disclosure has inhibitory effect against SARS-coronavirus 2 and variants thereof (e.g., Delta variant, Omicron variant, etc.), it can be usefully used for prevention or treatment of infection by SARS-coronavirus 2 or variants thereof.

## Description

### [Technical Field]

The present disclosure relates to a neutralizing antibody against SARS-coronavirus 2 or a variant virus thereof, or an antigen-binding fragment thereof.

### [Background Art]

Over the past two decades in the 21st century, there have been five pandemics such as SARS, swine flu, MERS, Ebola and coronavirus 2019. Experts predict that the period of outbreak of new pandemics will be reduced from 10 years to 3 years or shorter (Zhou, P. et al. A pneumonia outbreak associated with a new coronavirus of probable bat origin. Nature 579, 270-273 (2020).

Especially, major countries have failed in preventive measures against coronavirus 2019 and variants thereof in the early stage and have exposed problems in their response systems against infectious diseases. The US and major European countries have recorded large numbers of deaths and high mortalities. The potential global economic loss in 2020 due to the spread of coronavirus 2019 is expected to reach from 5 trillion and 800 billion dollars (about 7.13 quadrillion won) to 8 trillion and 800 dollars (10 quadrillion won). It is predicted that Asia will count for about 30% of the global loss with up to 2 trillion and 500 billion dollars (reported by the Asian Development Bank (ADB) in 2020, https://m.newspim.com/news/view/20200515000682).

Since the first outbreak of coronavirus 2019 or SARS-CoV2 in Wuhan, China, many variants (Alpha, Beta, Gamma, Delta, Omicron, etc.) are emerging. As of 2022, new variants of Omicron with further enhanced transmissibility such as BA.2 (Stealth Omicron), BA.2.12.1, BA.4, BA.5, etc. are threatening the mankind.

However, the development of antibodies capable of neutralizing the Alpha, Delta and Omicron variants is retarded and the market demand is increasing rapidly.

Throughout the present specification, a number of papers and patent documents are referred to and their citations are indicated. The disclosed contents of the papers and patent documents are included herein by reference in their entirety, so that the related art and the contents of the present disclosure could be described more clearly.

### [Disclosure]

### [Technical Problem]

The inventors of the present disclosure have made consistent efforts to develop an antibody having inhibitory effect against various coronavirus variants including the Delta and Omicron variants. As a result of the efforts, they have screened 16 candidate antibodies having similar CDR sequences and have completed the present disclosure by identifying that some of the antibodies have excellent neutralizing effect not only for the Delta variant but also for the Omicron variant.

The present disclosure is directed to providing a neutralizing antibody against SARS-coronavirus 2 (SARS-CoV2) or a variant virus thereof, or an antigen-binding fragment thereof.

The present disclosure is also directed to providing a nucleic acid molecule encoding the antibody or an antigen-binding fragment thereof.

The present disclosure is also directed to providing a vector including the nucleic acid molecule.

The present disclosure is also directed to providing a cell including the vector.

The present disclosure is also directed to providing a pharmaceutical composition for preventing or treating infection by SARS-coronavirus 2 (SARS-CoV2) or a variant virus thereof, which contains the antibody or an antigen-binding fragment thereof, a nucleic acid molecule encoding the antibody or an antigen-binding fragment thereof, or a vector including the nucleic acid molecule as an active ingredient.

The present disclosure is also directed to providing a method for preventing or treating infection by SARS-coronavirus 2 (SARS-CoV2) or a variant virus thereof, which includes a step of administering a pharmaceutically effective amount of the antibody or an antigen-binding fragment thereof, a nucleic acid molecule encoding the antibody or an antigen-binding fragment thereof, or a vector including the nucleic acid molecule to a subject.

The present disclosure is also directed to providing a use of the antibody or an antigen-binding fragment thereof, a nucleic acid molecule encoding the antibody or an antigen-binding fragment thereof, or a vector including the nucleic acid molecule in therapy.

The present disclosure is also directed to providing a composition for diagnosing infection by SARS-coronavirus 2 (SARS-CoV2) or a variant virus thereof, which contains the antibody or an antigen-binding fragment thereof, a nucleic acid molecule encoding the antibody or an antigen-binding fragment thereof, or a vector including the nucleic acid molecule as an active ingredient.

Other purposes and advantages of the present disclosure become more apparent by the following detailed description, claims and drawings.

### [Technical Solution]

In an aspect, the present disclosure provides a neutralizing antibody against SARS-coronavirus 2 (SARS-CoV2) or a variant virus thereof, or an antigen-binding fragment thereof, wherein
the antibody or an antigen-binding fragment thereof includes light chain CDR1, CDR2 and CDR3 and heavy chain CDR1, CDR2 and CDR3, and
the light chain CDR1 is represented by an amino acid sequence of General Formula 1; the light chain CDR2 is represented by an amino acid sequence of General Formula 2; the light chain CDR3 is represented by an amino acid sequence of General Formula 3; the heavy chain CDR1 is represented by an amino acid sequence of General Formula 4; the heavy chain CDR2 is represented by an amino acid sequence of General Formula 5; and the heavy chain CDR3 is represented by an amino acid sequence of General Formula 6, General Formula 7 or General Formula 8:

   **General Formula 1** X₁₁-G-S-S-S-N-I-G-X₁₂-X₁₃-X₁₄-V-X₁₅
wherein X₁₁ is S or T; X₁₂ is S or N; X₁₃ is N or T; X₁₄ is Y, D, A, N, T or S; and X₁₅ is S, T, Y or N;

   **General Formula 2** X₂₁-X₂₂-X₂₃-X₂₄
wherein X₂₁ is S, A or D; X₂₂ is D or N; X₂₃ is N or S; and X₂₄ is N, K, H or Q;

   **General Formula 3** X₃₁-X₃₂-W-D-X₃₃-S-L-X₃₄-X₃₅
wherein X₃₁ is A or G; X₃₂ is T, A or S; X₃₃ is A, Y or D; X₃₄ is S or N; and X₃₅ is A or G;

   **General Formula 4** X₄₁-Y-X₄₂-M-S
wherein X₄₁ is N, D or G; and X₄₂ is A, S, Y or D;

   **General Formula 5** X₅₁-I-X₅₂-X₅₃-X₅₄-X₅₅-X₅₆-X₅₇-X₅₈-X₅₉
wherein X₅₁ is W, A, G, E, V, M or L; X₅₂ is Y or S; X₅₃ is S, H, Y or P; X₂₄ is N, D, S, H or G; X₅₅ is D, S, G or N; X₅₆ is G or S; X₅₇ is N or S; X₅₈ is N, K, T or I; and X₅₉ is nonexistent or T;

   **General Formula 6** X601-X602-X603-X604-X605-X606-X607-X608-X609-X610-X611-X612-X613-X614-D-A-M-D-V
wherein X₆₀₁ is R or K; X₆₀₂ is F, V or A; X₆₀₃ is P, T or I; X₆₀₄ is V, G or L; X₆₀₅ is P, T or R; X₆₀₆ is S or C; X₆₀₇ is W, H or I; X₆₀₈ is R, S or P; X₆₀₉ is N, G or L; X₆₁₀ is T, S or G; X₆₁₁ is W or C; X₆₁₂ is S or Y; X₆₁₃ is S or Y; and X₆₁₄ is Y, S or A;

   **General Formula 7** X₇₀₁ -X₇₀₂-X₇₀₃-X₇₀₄-X₇₀₅-X₇₀₆-X₇₀₇-X₇₀₈-X₇₀₉-X₇₁₀-F-D-V
wherein X₇₀₁ is R or K; X₇₀₂ is D or G; X₇₀₃ is P, A or V, X₇₀₄ is F, W or P; X₇₀₅ is P, T or E; X₇₀₆ is G, L or I; X₇₀₇ is R, H or A; X₇₀₈ is S or P; X₇₀₉ is S, For P; and X₇₁₀ is N, S or R; and

   **General Formula 8** X₈₁-X₈₂-X₈₃-X₈₄-X₈₅-F-D-Y
wherein X₈₁ is R or K; X₈₂ is P, D, Y, F, N, S or G; X₈₃ is K, F, G, Q, D, L, H, P, A or T; X₈₄ is T, G, N, W, P or F; and X₈₅ is S, P, T, D, V, G or W.

In the present specification, the term "antibody" refers to a protein molecule that acts as a receptor capable of specifically recognizing an antigen, and it includes an immunoglobulin molecule that is immunologically reactive with a particular antigen. The antibody includes a polyclonal antibody and a monoclonal antibody.

The antibody includes not only a whole full-length antibody but also an antigen-binding fragment of the antibody molecule. A whole antibody has a structure with two full-length light chains and two full-length heavy chains, in which each light chain is linked to a heavy chain by ad disulfide bond. The constant region of the heavy chain has gamma (γ), mu (µ), alpha (α), Delta (δ) and epsilon (ε) types, with the subclasses of gamma 1 (γ1 ), gamma 2 (γ2), gamma 3 (γ3), gamma 4 (γ4), alpha 1 (α1) and alpha 2 (α2). The constant region of the light chain has kappa (κ) and lambda (λ) types.

In the present specification, the term " antigen-binding fragment of an antibody" refers to a fragment of the whole antibody molecule that specifically recognizes an antigen and has the function of antigen-antibody binding, and includes a single-domain antibody (sdAb), a single-chain variable fragment (scFv), Fab, F(ab'), F(ab')₂, Fv, etc. The fragment includes, for example, a monovalent fragment (Fab fragment) consisting of V_{L}, V_{H}, C_{K} (or C_{L}) and C_{H}1 domains; a bivalent fragment (F(ab')₂ fragment) including two Fab fragments linked by a disulfide bridge at the hinge region; an Fd fragment consisting of V_{H} and C_{H}1 domains; an Fv fragment consisting of V_{L} and V_{H} domains of a single arm of an antibody and disulfide-linked Fv (sdFv); a dAb fragment consisting of a V_{H} domain; and a combination of two or more separated complementarity-determining regions (CDRs) optionally kinked by a linker. Furthermore, the V_{L} and V_{H} regions of scFv may be linked by a linker to form a single protein chain. Such a single-chain antibody is also included in the antibody fragment. In addition, the antibody or antibody fragment includes a tetrameric antibody including two heavy chain molecules and two light chain molecules; an antibody light chain monomer; an antibody heavy chain monomer; an antibody light chain dimer, antibody heavy chain dimer; an intrabody; a monovalent antibody; a camel antibody; and a single-domain antibody (sdAb).

Fv is a minimal antibody fragment having a heavy chain variable region and a light chain variable region. Recombinant technologies for producing Fv fragments are disclosed in International Publication Nos. WO 88/10649, WO 88/106630, WO 88/07085, WO 88/07086 and WO 88/09344. Two-chain Fv is a fragment wherein a heavy chain variable region and a light chain variable region are linked by a noncovalent bond, and single-chain Fv is a fragment wherein the variable region of a heavy chain and the variable region of a light chain are linked by a covalent bond via a peptide linker or are linked directly at the C-terminal to form a dimer-like structure like the two-chain Fv. Such antibody fragments may be obtained using proteases (e.g., Fab can be obtained by restriction-cleaving the whole antibody with papain, and F(ab')₂ fragment can be obtained by cleaving with pepsin), and may be prepared by genetic recombination techniques.

In the present specification, the term "heavy chain" encompasses both a full-length heavy chain which includes a variable region domain V_{H} containing an amino acid sequence having a sufficient variable region sequence for imparting specificity to an antigen and three constant region domains C_{H}1, C_{H}2 and C_{H}3, and a fragment thereof. And, in the present specification, the term "light chain" encompasses both a full-length light chain which includes a variable region domain V_{L} containing an amino acid sequence having a sufficient variable region sequence for imparting specificity to an antigen and a constant region domain C_{K} (or C_{L}), and a fragment thereof.

In the present specification, the term "CDR (complementarity-determining region)" refers to an amino acid sequence found in the hypervariable region of a heavy chain and a light chain of an immunoglobulin (Kabat et al. Sequences of Proteins of Immunological Interest, 4th Ed., U.S. Department of Health and Human Services, National Institutes of Health (1987)). The heavy chain (HCDR1, HCDR2 and HCDR3) and light chain (LCDR1, LCDR2 and LCDR3) include three CDRs, respectively. The CDRs provide an essential contact residue for binding of an antibody to an antigen or an epitope. The CDRs are interspersed with regions that are more conserved, termed framework regions (FRs). Each V_{H} and V_{L} is composed of three CDRs and four FRs arranged from the amino-terminal to the carboxyl-terminal in the following order: FR1, CDR1, FR2, CDR2, FR3, CDR3 and FR4.

The antibody or antibody fragment of the present disclosure encompasses variants having conservative amino acid substitutions at the CDR regions. For example, additional changes in the amino acid sequence of an antibody can be made to improve the binding affinity of the antibody or other biological characteristics such as half-life and biocompatibility. Considering these variations having biologically equivalent activity, it is interpreted that the antibody of the present disclosure or a nucleic acid molecule encoding the same includes those having sequences exhibiting substantial identity to those listed in the sequence text list. The substantial identity means that the sequence of the present disclosure exhibits at least 60%, specifically 70%, more specifically 80%, further more specifically 90%, of homology when the sequence and any other sequence are aligned to match as much as possible and analyzed by an algorithm commonly used in the art. The alignment method for sequence comparison is known in the art. Various methods and algorithms for the alignment are disclosed in Smith and Waterman, Adv. Appl. Math. (1981) 2: 482 Needleman and Wunsch, J. Mol. Bio. (1970) 48: 443; Pearson and Lipman, Methods in Mol. Biol. (1988) 24: 307-31; Higgins and Sharp, Gene (1988) 73: 237-44; Higgins and Sharp, CABIOS (1989) 5: 151-3; Corpet et al. Nuc. Acids Res. (1988) 16: 10881-90; Huang et al. Comp. Appl. BioSci. (1992) 8: 155-65 and Pearson et al. Meth. Mol. Biol. (1994) 24: 307-31.

Coronavirus is generally known as the cause of cold symptoms in birds and mammals. It caused severe acute respiratory syndrome (SARS) and Middle East respiratory syndrome (MERS) in the past and is drawing attentions recently due to coronavirus disease 2019 (COVID-19) that broke out in Whuan, China.

The virus that caused the coronavirus disease 2019 is SARS-CoV-2 which is a variant of coronavirus. Unlike the previous coronaviruses, SARS-CoV-2 has mutation in the 'spike' protein for binding to respiratory epithelial cells.

The variant viruses include various variant viruses that prevailed in the prevailed, such as Alpha, Beta, Gamma, Delta, Omicron, Lambda, Mu, etc., or are likely to prevail in the future. Specifically, the variant virus may be Delta or Omicron variant virus, although not being limited thereto.

According to a specific exemplary embodiment of the present disclosure, the variant virus is Omicron variant virus.

According to a specific exemplary embodiment of the present disclosure, in the light chain CDR1 of the antibody or the antigen-binding fragment thereof, X₁₄ and X₁₅ are S or T.

According to a specific exemplary embodiment of the present disclosure, in the light chain CDR2 of the antibody or the antigen-binding fragment thereof, X₂₁ is A or D; X₂₂ is D; and X₂₄ is N, H or Q.

According to a specific exemplary embodiment of the present disclosure, in the light chain CDR3 of the antibody or the antigen-binding fragment thereof, X₃₂ is A; X₃₃ is D; and X₃₄ is S.

According to a specific exemplary embodiment of the present disclosure, in the heavy chain CDR1 of the antibody or the antigen-binding fragment thereof, X₄₂ is A, Y or D.

According to a specific exemplary embodiment of the present disclosure, in the heavy chain CDR2 of the antibody or the antigen-binding fragment thereof, X₅₁ is G or L; X₅₃ is H, Y or P; X₅₄ is N or G; X₅₅ is S, G or N; and X₅₈ is K, T or N.

According to a specific exemplary embodiment of the present disclosure, the heavy chain CDR3 of the antibody or the antigen-binding fragment thereof is an amino acid sequence of General Formula 7 and wherein X₇₀₂ is D; X₇₀₃ is P or A, X₇₀₄ is F or W; X₇₀₅ is P or T; X₇₀₆ is G or L; X₇₀₇ is R or H; X₇₀₉ is S or F; and X₇₁₀ is N or S.

According to a specific exemplary embodiment of the present disclosure, the heavy chain CDR3 of the antibody or the antigen-binding fragment thereof is an amino acid sequence of General Formula 8 and wherein X₈₁ is K; X₈₂ is Y; X₈₃ is Q; X₈₄ is W; and X₈₅ is D.

According to a specific exemplary embodiment of the present disclosure, the antibody or the antigen-binding fragment thereof includes light chain CDR1, CDR2 and CDR3 and heavy chain CDR1, CDR2 and CDR3, wherein
(1) the light chain CDR1 is represented by an amino acid sequence of SEQ ID NO 9, the light chain CDR2 is represented by an amino acid sequence of SEQ ID NO 20, the light chain CDR3 is represented by an amino acid sequence of SEQ ID NO 28, the heavy chain CDR1 is represented by an amino acid sequence of SEQ ID NO 41, the heavy chain CDR2 is represented by an amino acid sequence of SEQ ID NO 48 and the heavy chain CDR3 is represented by an amino acid sequence of SEQ ID NO 64;
(2) the light chain CDR1 is represented by an amino acid sequence of SEQ ID NO 10, the light chain CDR2 is represented by an amino acid sequence of SEQ ID NO 21, the light chain CDR3 is represented by an amino acid sequence of SEQ ID NO 29, the heavy chain CDR1 is represented by an amino acid sequence of SEQ ID NO 42, the heavy chain CDR2 is represented by an amino acid sequence of SEQ ID NO 49 and the heavy chain CDR3 is represented by an amino acid sequence of SEQ ID NO 65;
(3) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 41, the heavy chain CDR2 is represented by an amino acid sequence of SEQ ID NO 50 and the heavy chain CDR3 is represented by an amino acid sequence of SEQ ID NO 66;
(4) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 41, the heavy chain CDR2 is represented by an amino acid sequence of SEQ ID NO 51 and the heavy chain CDR3 is represented by an amino acid sequence of SEQ ID NO 67;
(5) the light chain CDR1 is represented by an amino acid sequence of SEQ ID NO 12, the light chain CDR2 is represented by an amino acid sequence of SEQ ID NO 23, the light chain CDR3 is represented by an amino acid sequence of SEQ ID NO 31, the heavy chain CDR1 is represented by an amino acid sequence of SEQ ID NO 43, the heavy chain CDR2 is represented by an amino acid sequence of SEQ ID NO 52 and the heavy chain CDR3 is represented by an amino acid sequence of SEQ ID NO 68;
(6) the light chain CDR1 is represented by an amino acid sequence of SEQ ID NO 13, the light chain CDR2 is represented by an amino acid sequence of SEQ ID NO 24, the light chain CDR3 is represented by an amino acid sequence of SEQ ID NO 32, the heavy chain CDR1 is represented by an amino acid sequence of SEQ ID NO 43, the heavy chain CDR2 is represented by an amino acid sequence of SEQ ID NO 53 and the heavy chain CDR3 is represented by an amino acid sequence of SEQ ID NO 69;
(7) the light chain CDR1 is represented by an amino acid sequence of SEQ ID NO 14, the light chain CDR2 is represented by an amino acid sequence of SEQ ID NO 24, the light chain CDR3 is represented by an amino acid sequence of SEQ ID NO 33, the heavy chain CDR1 is represented by an amino acid sequence of SEQ ID NO 42, the heavy chain CDR2 is represented by an amino acid sequence of SEQ ID NO 54 and the heavy chain CDR3 is represented by an amino acid sequence of SEQ ID NO 70;
(8) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 34, the heavy chain CDR1 is represented by an amino acid sequence of SEQ ID NO 41, the heavy chain CDR2 is represented by an amino acid sequence of SEQ ID NO 55 and the heavy chain CDR3 is represented by an amino acid sequence of SEQ ID NO 71;
(9) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 35, the heavy chain CDR1 is represented by an amino acid sequence of SEQ ID NO 44, the heavy chain CDR2 is represented by an amino acid sequence of SEQ ID NO 56 and the heavy chain CDR3 is represented by an amino acid sequence of SEQ ID NO 72;
(10) the light chain CDR1 is represented by an amino acid sequence of SEQ ID NO 15, the light chain CDR2 is represented by an amino acid sequence of SEQ ID NO 25, the light chain CDR3 is represented by an amino acid sequence of SEQ ID NO 36, the heavy chain CDR1 is represented by an amino acid sequence of SEQ ID NO 45, the heavy chain CDR2 is represented by an amino acid sequence of SEQ ID NO 57 and the heavy chain CDR3 is represented by an amino acid sequence of SEQ ID NO 73;
(11) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 42, the heavy chain CDR2 is represented by an amino acid sequence of SEQ ID NO 58 and the heavy chain CDR3 is represented by an amino acid sequence of SEQ ID NO 74;
(12) the light chain CDR1 is represented by an amino acid sequence of SEQ ID NO 16, the light chain CDR2 is represented by an amino acid sequence of SEQ ID NO 24, the light chain CDR3 is represented by an amino acid sequence of SEQ ID NO 37, the heavy chain CDR1 is represented by an amino acid sequence of SEQ ID NO 41, the heavy chain CDR2 is represented by an amino acid sequence of SEQ ID NO 59 and the heavy chain CDR3 is represented by an amino acid sequence of SEQ ID NO 75;
(13) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 46, the heavy chain CDR2 is represented by an amino acid sequence of SEQ ID NO 60 and the heavy chain CDR3 is represented by an amino acid sequence of SEQ ID NO 76;
(14) the light chain CDR1 is represented by an amino acid sequence of SEQ ID NO 17, the light chain CDR2 is represented by an amino acid sequence of SEQ ID NO 26, the light chain CDR3 is represented by an amino acid sequence of SEQ ID NO 38, the heavy chain CDR1 is represented by an amino acid sequence of SEQ ID NO 46, the heavy chain CDR2 is represented by an amino acid sequence of SEQ ID NO 61 and the heavy chain CDR3 is represented by an amino acid sequence of SEQ ID NO 77;
(15) the light chain CDR1 is represented by an amino acid sequence of SEQ ID NO 18, the light chain CDR2 is represented by an amino acid sequence of SEQ ID NO 27, the light chain CDR3 is represented by an amino acid sequence of SEQ ID NO 39, the heavy chain CDR1 is represented by an amino acid sequence of SEQ ID NO 47, the heavy chain CDR2 is represented by an amino acid sequence of SEQ ID NO 62 and the heavy chain CDR3 is represented by an amino acid sequence of SEQ ID NO 78; or
(16) the light chain CDR1 is represented by an amino acid sequence of SEQ ID NO 19, the light chain CDR2 is represented by an amino acid sequence of SEQ ID NO 23, the light chain CDR3 is represented by an amino acid sequence of SEQ ID NO 40, the heavy chain CDR1 is represented by an amino acid sequence of SEQ ID NO 41, the heavy chain CDR2 is represented by an amino acid sequence of SEQ ID NO 63 and the heavy chain CDR3 is represented by an amino acid sequence of SEQ ID NO 79.

The antibody or the antigen-binding fragment thereof of the present disclosure may include light chain FR1, FR2, FR3 and FR4 and/or heavy chain FR1, FR2, FR3 and FR4.

According to a specific exemplary embodiment of the present disclosure, the light chain FR1 may be represented by an amino acid sequence of SEQ ID NO 1, the light chain FR2 may be represented by an amino acid sequence of SEQ ID NO 2 the, light chain FR3 may be represented by an amino acid sequence of SEQ ID NO 3, the light chain FR4 may be represented by an amino acid sequence of SEQ ID NO 4, the heavy chain FR1 may be represented by an amino acid sequence of SEQ ID NO 5, the heavy chain FR2 may be represented by an amino acid sequence of SEQ ID NO 6, the heavy chain FR3 may be represented by an amino acid sequence of SEQ ID NO 7 and the heavy chain FR4 may be represented by an amino acid sequence of SEQ ID NO 8.

In another aspect, the present disclosure provides a nucleic acid molecule encoding the antibody or the antigen-binding fragment thereof.

In the present specification, the term "nucleic acid molecule" encompasses DNA (gDNA and cDNA) and RNA molecules. A nucleotide which is the basic component in the nucleic acid molecule includes not only a natural nucleotide but also an analogue with a modified sugar or base moiety (Scheit, Nucleotide Analogs, John Wiley, New York (1980); Uhlman and Peyman, Chemical Reviews, (1990) 90: 543-584). The sequence of the nucleic acid molecule encoding the heavy chain and light chain variable regions of the present disclosure may be modified. The modification includes addition, deletion or non-conservative or conservative substitution of the nucleotide.

The nucleic acid molecule of the present disclosure also includes a nucleic acid molecule having a substantial nucleotide sequence identity to the nucleotide sequences described above. The substantial identity means that the nucleotide sequence of the present disclosure exhibits at least 80%, specifically at least 90%, more specifically at least 95%, of homology when the sequence and any other sequence are aligned to match as much as possible and analyzed by an algorithm commonly used in the art.

In another aspect, the present disclosure provides a vector including the nucleic acid molecule encoding the antibody or the antigen-binding fragment thereof of the present disclosure.

In the present specification, the term "vector" refers to a carrier that can insert the polynucleotide (nucleic acid) sequence into a cell capable of replicating the polynucleotide sequence. The polynucleotide sequence may be exogenous or heterologous. The vector may be a plasmid, a cosmid vector or a viral vector (retroviral, adenoviral or adeno-associated viral vector), although not being limited thereto. Those skilled in the art can construct the vector by a standard recombinant technique (Maniatis, et al., Molecular Cloning, A Laboratory Manual, Cold Spring Harbor Press, Cold Spring Harbor, N.Y., 1988; and Ausubel et al., Current Protocols in Molecular Biology, John, Wiley & Sons, Inc., NY, 1994).

In the present specification, the term "expression vector" refers to a vector including a nucleotide sequence that encodes at least a part of the transcribed gene product. In some cases, a RNA molecule is translated into a protein, a polypeptide or a peptide thereafter. The expression vector may include various regulatory sequences. In addition to regulatory sequences that regulate transcription and translation, the vector and the expression vector may also include nucleic acid sequences that provide other functions.

In another aspect, the present disclosure provides a cell including the vector described above.

In the present specification, the term "cell" includes a eukaryotic cell and a prokaryotic cell, and refers to any transformable cell capable of replicating the vector or expressing a gene encoded by the vector. The cell may be transfected, transduced or transformed by the vector, which means a process whereby an exogenous polynucleotide (nucleic acid molecule) is delivered or introduced into a host cell. In the present specification, the term "transformation" encompasses transfection and transduction.

Specifically, the (host) cell of the present disclosure may be an insect cell or a mammalian cell, although not being limited thereto. More specifically, the insect cell may be an Sf9 cell, and the mammalian cell may be an HEK293 cell, a HeLa cell, an ARPE-19 cell, an RPE-1 cell, a HepG2 cell, a Hep3B cell, a Huh-7 cell, a C8D1a cell, a Neuro2A cell, a CHO cell, an MES13 cell, a BHK-21 cell, a COS7 cell, a COP5 cell, an A549 cell, an MCF-7 cell, an HC70 cell, an HCC1428 cell, a BT-549 cell, a PC3 cell, an LNCaP cell, a Capan-1 cell, a Panc-1 cell, a MIA PaCa-2 cell, an SW480 cell, an HCT166 cell, a LoVo cell, an A172 cell, an MKN-45 cell, an MKN-74 cell, a Kato-III cell, an NCI-N87 cell, an HT-144 cell, an SK-MEL-2 cell, an SH-SY5Y cell, a C6 cell, an HT-22 cell, a PC-12 cell, an NIH3T3 cell, etc.

Specifically, the host cell of the present disclosure is an isolated host cell.

In another aspect, the present disclosure provides a pharmaceutical composition for preventing or treating infection by SARS-coronavirus 2 (SARS-CoV2) or a variant virus thereof, which contains the antibody or the antigen-binding fragment thereof of the present disclosure, a nucleic acid molecule encoding the antibody or the antigen-binding fragment thereof, or a vector including the nucleic acid molecule as an active ingredient.

In another aspect, the present disclosure provides a method for preventing or treating infection by SARS-coronavirus 2 (SARS-CoV2) or a variant virus thereof, which includes a step of administering a pharmaceutically effective amount of the antigen-binding fragment thereof of the present disclosure, a nucleic acid molecule encoding the antibody or the antigen-binding fragment thereof, or a vector including the nucleic acid molecule to a subject.

In another aspect, the present disclosure provides a use of the antigen-binding fragment thereof of the present disclosure, a nucleic acid molecule encoding the antibody or the antigen-binding fragment thereof, or a vector including the nucleic acid molecule in therapy.

Specifically, the use in therapy is a use for treating infection by SARS-coronavirus 2 (SARS-CoV2) or a variant virus thereof.

In the present specification, the term "prevention" refers to the prevention of the onset of a disease in a subject who has not been diagnosed with the disease but has the risk of having the disease.

In the present specification, the term "treatment" refers to the (a) suppression of the development of a disease, disorder or symptoms; (b) alleviation of the disease, disorder or symptoms; or (c) removal of the disease, disorder or symptoms. The composition of the present disclosure can be used as a composition for treating viral infection on its own or as a therapeutic adjuvant for improving the therapeutic response of another pharmaceutical ingredient. Therefore, in the present specification, the term "treatment" or "therapeutic agent" includes the meaning of "aid of treatment" or "therapeutic adjuvant".

In the present specification, the term "administration" or "administer" refers to the direct administration of a therapeutically effective amount of the composition of the present disclosure to a subject for formation of the same amount in the subject.

In the present specification, the term "pharmaceutically effective amount" or "therapeutically effective amount" means the amount of a pharmaceutical composition which is sufficient to provide the therapeutic or prophylactic effect of a pharmaceutical ingredient of the composition in a subject to which the composition is to be administered, and thus encompasses the meaning of "prophylactically effective amount".

In the present specification, the term "subject" includes human, mouse, rat, guinea pig, dog, cat, horse, cow, pig, monkey, chimpanzee, baboon or rhesus monkey without limitation. Specifically, the subject of the present disclosure is human.

According to a specific exemplary embodiment of the present disclosure, the pharmaceutical composition of the present disclosure contains a pharmaceutically acceptable carrier or excipient.

The pharmaceutical composition of the present disclosure may be prepared into a single-dose or multiple-dose form using a pharmaceutically acceptable carrier and/or excipient according to a method that can be easily carried out by those having ordinary knowledge in the art to which the present disclosure belongs.

The pharmaceutical composition according to the present disclosure may be formulated into various forms according to common methods. For example, it may be formulated into an oral formulation such as a powder, a granule, a tablet, a capsule, a suspension, an emulsion, a syrup, etc. or into a formulation for external application, a suppository or a sterilized solution for injection.

The composition of the present disclosure may contain one or more active ingredient known to have antiviral activity.

The pharmaceutical composition of the present disclosure may be administered orally or parenterally. Specifically, it may be administered parenterally, e.g., by intravenous injection, transdermal administration, subcutaneous injection, intramuscular injection, intravitreal injection, subretinal injection, suprachoroidal injection, eye drop administration, intracerebroventricular injection, intrathecal injection, intraamniotic injection, intraarterial injection, intraarticular injection, intracardiac injection, intracavernous injection, intracerebral injection, intracisternal injection, intracoronary injection, intracranial injection, intradural injection, epidural injection, intrahippocampal injection, intranasal injection, intraosseous injection, intraperitoneal injection, intrapleural injection, intraspinal injection, intrathoracic injection, intrathymic injection, intrauterine injection, intravaginal injection, intraventricular injection, intravesical injection, subconjunctival injection, intratumoral injection, topical injection, etc.

Formulations for the parenteral administration include a sterilized aqueous solution, a nonaqueous solution, a suspension, an emulsion, a lyophilized formulation and a suppository. As a solvent for the nonaqueous solution or the suspension, propylene glycol, polyethylene glycol, a vegetable oil such as olive oil, an injectable ester such as ethyl oleate, etc. may be used. As a base of the suppository, witepsol, macrogol, macrogol, Tween 61, cocoa butter, laurin butter, glycerogelatin, etc. may be used.

The administration dosage of the pharmaceutical composition of the present disclosure may vary depending on the formulation method of the pharmaceutical composition, administration method, administration time, administration route, etc., and may vary depending on various factors such as the response to be achieved with the administration of the pharmaceutical composition and the extent thereof, the subject as a target of the administration and the age, body weight, general health status, symptoms or degree of a disease, sex, diet or excretion rate thereof, other drug ingredients co-administered to the subject, and other similar factors well known in the medical field. Those having ordinary knowledge in the art can easily determine and describe an administration dosage effective for the desired treatment.

The administration route and administration method of the pharmaceutical composition of the present disclosure may be independent from each other and are not specially limited. Any administration route and administration method may be selected as long as the pharmaceutical composition can reach the desired site.

In another aspect, the present disclosure provides a composition for diagnosing infection by SARS-coronavirus 2 (SARS-CoV2) or a variant virus thereof, which contains a nucleic acid molecule encoding the antibody or the antigen-binding fragment thereof of the present disclosure, or a vector including the nucleic acid molecule as an active ingredient.

Since the antibody or the antigen-binding fragment and the SARS-coronavirus 2 (SARS-CoV2) or the variant virus thereof have been described above, description thereof will be omitted to avoid unnecessary redundancy.

In the present specification, the term "diagnosis" encompasses the determination of the susceptibility of a subject for a specific disease, determination of whether a subject currently has a specific disease and determination of a prognosis of a subject with a specific disease.

The antibody of the present disclosure which binds specifically to SARS-coronavirus 2 (SARS-CoV2) or a variant virus thereof can accurately determine the presence of the virus in a biological sample and can be usefully used to establish a therapeutic strategy in the early stage.

In the present specification, the term "composition for diagnosis" refers to a mixture or a device including the antibody of the present disclosure for determination of viral infection in a subject and it also may be expressed as a "kit for diagnosis".

In the present specification, the term "biological sample" includes a tissue, a cell, whole blood, serum, plasma, saliva, nasal discharge, urine, lymph, spinal fluid, a tissue (brain, skin, lymph node, spinal code, etc.) biopsy sample, a cell culture supernatant, a ruptured eukaryotic cell and a bacterial expression system, although not being limited thereto.

The detection of viruses in a biological sample may be performed by detection of the formation of an antigen-antibody complex through colorimetric method, electrochemical method, fluorometric method, luminometric method, particle counting method, visual assessment or scintillation counting method. In the present specification, the term "detection" refers to a series of process for determining the formation of the antigen-antibody complex. The detection may be carried out using various labels including an enzyme, a fluorophore, a ligand, a luminophore, a microparticle or a radioisotope.

The enzyme used as the detection label may include, for example, acetylcholinesterase, alkaline phosphatase, β-D-galactosidase, horseradish peroxidase and β-lactamase, the fluorophore may include fluorescein, Eu³⁺, Eu³⁺ chelate or cryptate, etc., the ligand may include a biotin derivative, etc., the luminophore may include an acridinium ester, an isoluminol derivative, etc., the microparticle may include colloidal gold, colored latex, tec., and the radioisotope may include ⁵⁷Co, ³H, ¹²⁵I, a ¹²⁵I-Bonton Hunter reagent, etc.

According to an exemplary embodiment of the present disclosure, the antigen-antibody complex may be detected by enzyme-linked immunosorbent assay (ELISA). The antibody of the present disclosure may have a detection label. When it has no detection label, it may be identified by treating with another antibody that can capture the antibody of the present disclosure and has a detection label.

### [Advantageous Effects]

The features and advantages of the present disclosure may be summarized as follows:
(i) The present disclosure provides a neutralizing antibody against SARS-coronavirus 2 (SARS-CoV2) or a variant virus thereof, or an antigen-binding fragment thereof.
(ii) Since the neutralizing antibody of the present disclosure has inhibitory effect against SARS-coronavirus 2 and variant viruses thereof (e.g., Delta variant, Omicron variant, etc.), it can be usefully used for prevention or treatment of infection by SARS-coronavirus 2 or variant viruses thereof.

### [Brief Description of Drawings]

FIG. 1 schematically shows a process of screening candidate antibodies through biopanning.
FIGS. 2A and 2B show a result of screening candidate neutralizing antibodies against SARS-CoV2 variant viruses by ELISA.
FIG. 3 shows a result of identifying the binding ability of 13 candidate neutralizing antibodies to antigens (SARS-CoV2, B1.617.2 and B.1.1.529) by ELISA.
FIG. 4 shows a result of measuring the binding ability of SKAI-DS10, DS63 and DS84 antibodies screened through antibody-antigen protein thermal shift analysis (PTS) to the RBD antigens of Delta variant and Omicron variant.
FIG. 5 schematically shows a process of constructing a pseudovirus system for verifying the neutralizing ability of screened antibodies.
FIG. 6 shows a result of verifying the neutralizing ability of candidate neutralizing antibodies using a pseudovirus.
FIG. 7 shows a result of verifying the neutralizing effect (EC₅₀ and EC₉₀) of 3 candidate antibodies against a pseudovirus at different concentrations.
FIG. 8 shows the EC₅₀ and EC₉₀ concentrations for neutralizing antibodies depending on viral variation.
FIG. 9 shows a result of verifying neutralizing effect and antibody-dependent enhancement (ADE) using a SARS-CoV2 surrogate virus neutralization test kit.
FIG. 10 shows a result of verifying the affinity of a SKAI-DS84 antibody by surface plasmon response analysis.

### [Best Mode]

Hereinafter, the present disclosure will be described in more detail through examples. The examples are only for describing the present disclosure more specifically and it will be obvious to those having ordinary knowledge in the art that the scope of the present disclosure is not limited by the examples.

### Examples

### 1. Screening of candidate antibodies through biopanning

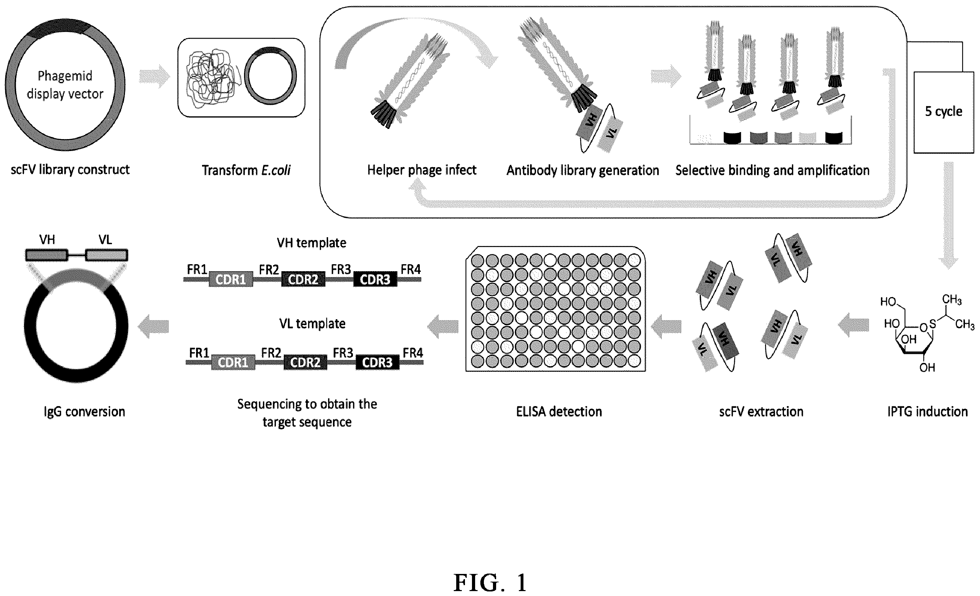

The phage display technique was used for development of neutralizing antibodies that inhibit variant viruses of coronavirus-19. 1x10¹⁰ scFvs acquired from Ewha Womans University were subjected to biopanning using an HA-tagged OPAL antibody library. After transforming ER2537 cells (New England Biolabs) with the library, they were cultured at 37 °C for 14 hours in SB medium (Difco^{tm}) containing 50 µg/mL carbenicillin and a helper phage. Biopanning was performed to screen scFvs that bind to SARS-CoV-2-B.1.617.2 (Delta variant) RBD. The SARS-CoV-2 Delta variant RBD was bound to epoxy magnetic beads and cultured with scFv-expressing phages. Then, the phages bound to the SARS-CoV-2 Delta variant RBD were eluted and new ER2537 cells were infected therewith. This process was repeated 5 times and candidate antibodies were screened by ELISA (FIG. 1).

### 2. Screening of candidate neutralizing antibodies against SARS-CoV2 variant virus by ELISA

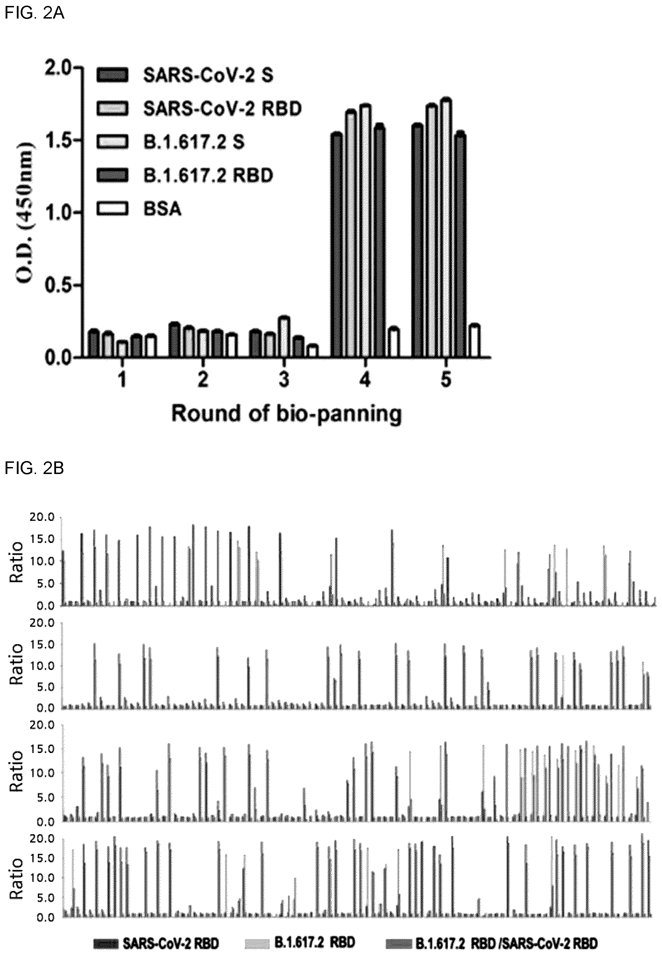

As a result of the five rounds of biopanning, it was identified that the scFvs were bound to both the S protein and RBD protein (Genscript) of SARS-CoV2 and Delta variant in the fourth and fifth rounds and antibody diversity was recovered. scFvs were expressed from 400 individual colonies recovered in the fourth and fifth rounds, and the degree of binding of the 400 scFvs was investigated by ELISA using SARS-CoV2 RBD, Delta variant RBD and Omicron variant RBD antigens. Finally, individual colonies exhibiting O.D. values (Delta RBD O.D. (red) / SARS-CoV2 RBD O.D.₄₅₀ (blue)) of 15 higher were screened as candidate antibodies (FIGS. 2A and 2B). As a result, 16 candidate neutralizing antibodies were screened, and their light chain and heavy chain CDR1, CDR2 and CDR3 antibody sequences were identified through genetic analysis (Tables 1 and 2).

**[Table 1]**

| **Light chain variable region** | | | | | | | |
|---|---|---|---|---|---|---|---|
| Seri al | FR1: | CDR1: | FR2: | CDR2: | FR3: | CDR3: | FR4: |
| SKA I-DS1 | QSVLTQPPSAS GTPGRRVTISC (SEQ ID NO: 1) | TGSSSNI GSNYVS (SEQ ID NO: 9) | WYQQLPG TAPKLLIY (SEQ ID NO: 2) | **SDSN** (SEQ ID NO: 20) | RPSGVPDRFS GSKSGTSASL AISGLRSEDEA DYYC (SEQ ID NO: 3) | GTWDA SLSG (SEQ ID NO: 28) | YVFGGG TKLTVL (SEQ ID NO: 4) |
| SKA I- | QSVLTQPPSAS GTPGQRVTISC | TGSSSNI GSNDVT( | WYQQLPG TAPKLLIY | **ADSK** (SEQ ID NO: | RPSGVPDRFS GSKSGTSASL | GAWDY SLSA | YVFGGG TKLTVL |
| DS3 | | SEQ ID NO: 10) | | 21) | AISGLRSEDEA DYYC | (SEQ ID NO: 29) | |
| SKA I-DS7 | QSVLTQPPSAS GTPGQRVTISC | SGSSSNI GSNAVS( SEQ ID NO: 11) | WYQQLPG TAPKLLIY | **DNNH** (SEQ ID NO: 22) | RPSGVPDRFS GSKSGTSASL AISGLRSEDEA DYYC | GSWDD SLNA (SEQ ID NO: 30) | YVFGGG TKLTVL |
| SKA I-DS1 0 | QSVLTQPPSAS GTPGQRVTISC | SGSSSNI GSNAVS( SEQ ID NO: 11) | WYQQLPG TAPKLLIY | **DNNH** (SEQ ID NO: 22) | RPSGVPDRFS GSKSGTSASL AISGLRSEDEA DYYC | GSWDD SLNA (SEQ ID NO: 30) | YVFGGG TKLTVL |
| SKA I-DS1 4 | QSVLTQPPSAS GTPGQRVTISC | SGSSSNI GSTNVY( SEQ ID NO: 12) | WYQQLPG TAPKLLIY | **SDNK** (SEQ ID NO: 23) | RPSGVPDRFS GSKSGTSASL AISGLRSEDEA DYYC | ASWDA SLNG (SEQ ID NO: 31) | YVFGGG TKLTVL |
| SKA I-DS1 5 | QSVLTQPPSAS GTPGQRVTISC | SGSSSNI GSNDVN( SEQ ID NO: 13) | WYQQLPG TAPKLLIY | **DDSN** (SEQ ID NO: 24) | RPSGVPDRFS GSKSGTSASL AISGLRSEDEA DYYC | GAWDD SLSG (SEQ ID NO: 32) | YVFGGG TKLTVL |
| SKA I-DS1 6 | QSVLTQPPSAS GTPGQRVTISC | SGSSSNI GSNYVY( SEQ ID NO: 14) | WYQQLPG TAPKLLIY | **DDSN** (SEQ ID NO: 24) | RPSGVPGRFS GSKSGTSASL AISGLRSEDEA DYYC | GTWDY SLNG (SEQ ID NO: 33) | YVFGGG TKLTVL |
| SKA I-DS1 7 | QSVLTQPPSAS GTPGQRVTISC | SGSSSNI GSNAVS( SEQ ID NO: 11) | WYQQLPG TAPKLLIY | **DNNH** (SEQ ID NO: 22) | RPSGVPDRFS GSKSGTSASL AISGLRSEDEA DYYC | GSWDD SLNA (SEQ ID NO: 34) | YVFGGG TKLTVL |
| SKA I-DS4 | QSVLTQPPSAS GTPGQRVTISC | SGSSSNI GSNAVS( SEQ ID | WYQQLPG TAPKLLIY | **DNNH** (SEQ ID NO: 22) | RPSGVPDRFS GSKSGTSASL AISGLRSEDEA | GSWDD SLNG (SEQ ID | YVFGGG TKLTVL |
| 2 | | NO: 11) | | | DYYC | NO: 35) | |
| SKA I-DS6 3 | QSVLTQPPSAS GTPGQRVTISC | SGSSSNI GSNTVS( SEQ ID NO: 15) | WYQQLPG TAPKLLIY | **ADNN** (SEQ ID NO: 25) | RPSGVPDRFS GSKSGTSASL AISGLRSEDEA DYYC | AAWDD SLSA (SEQ ID NO: 36) | YVFGGG TKLTVL |
| SKA I-DS6 4 | QSVLTQPPSAS GTPGQRVTISC | SGSSSNI GSNAVS( SEQ ID NO: 11) | WYQQLPG TAPKLLIY | **DNNH** (SEQ ID NO: 22) | RPSGVPDRFS GSKSGTSASL AISGLRSEDEA DYYC | GSWDD SLNA (SEQ ID NO: 30) | YVFGGG TKLTVL |
| SKA I-DS6 6 | QSVLTQPPSAS GTPGQRVTISC | TGSSSNI GSNAVY( SEQ ID NO: 16) | WYQQLPG TAPKLLIY | **DDSN** (SEQ ID NO: 24) | RPSGVPDRFS GSKSGTSASL AISGLRSEDEA DYYC | GSWDD SLSA (SEQ ID NO: 37) | YVFGGG TKLTVL |
| SKA I-DS7 7 | QSVLTQPPSAS GTPGQRVTISC | SGSSSNI GSNAVS( SEQ ID NO: 11) | WYQQLPG TAPKLLIY | **DNNH** (SEQ ID NO: 22) | RPSGVPDRFS GSKSGTSASL AISGLRSEDEA DYYC | GSWDD SLNA (SEQ ID NO: 30) | YVFGGG TKLTVL |
| SKA I-DS8 4 | QSVLTQPPSAS GTPGQRVTISC | TGSSSNI GNNSVT( SEQ ID NO: 17) | WYQQLPG TAPKLLIY | **ADSH** (SEQ ID NO: 26) | RPSGVPDRFS GSKSGTSASL AISGLRSEDEA DYYC | GAWDD SLSG (SEQ ID NO: 38) | YVFGGG TKLTVL |
| SKA I-DS9 5 | QSVLTQPPSAS GTPGQRVTISC | TGSSSNI GSNSVT( SEQ ID NO: 18) | WYQQLPG TAPKLLIY | **DDNQ** (SEQ ID NO: 27) | RPSGVPDRFS GSKSGTSASL AISGLRSEDEA DYYC | AAWDD SLSG (SEQ ID NO: 39) | YVFGGG TKLTVL |
| SKA I-DS1 04 | QSVLTQPPSAS GTPGQRVTISC | TGSSSNI GNNTVN( SEQ ID NO: 19) | WYQQLPG TAPKLLIY | **SDNK** (SEQ ID NO: 23) | RPSGVPDRFS GSKSGTSASL AISGLRSEDEA DYYC | AAWDA SLSA (SEQ ID NO: 40) | YVFGGG TKLTVL |

Light chain variable regions of SARS-CoV2 variant virus neutralizing antibodies

**[Table 2]**

| **Heavy chain variable region** | | | | | | | |
|---|---|---|---|---|---|---|---|
| Seri al | FR1: | CDR1: | FR2: | CDR2: | FR3: | CDR3: | FR4: |
| SKA I-DS1 | EVQLLESG GGLVQTGG SLRLSCAAS GFTFS(SEQ ID NO: 5) | **DYAMS** (SEQ ID NO: 41) | WVRQAPGK GLEWVS (SEQ ID NO: 6) | WIYSNDGN K (SEQ ID NO: 48) | YYADSVKG RFTISRDNS KNTLYLQM NSLRAEDT AVYYCA (SEQ ID NO: 7) | RFPVPSWR NTWSSYDA MDV (SEQ ID NO: 64) | WGQGTLVT V (SEQ ID NO: 8) |
| SKA I-DS3 | EVQLLESG GGLVQPGG SLRLSCAAS GFTFS | **DYSMS(SE** Q ID NO: 42) | WVRQAPGK GLEWVS | **AISSDSSST** (SEQ ID NO: 49) | YYADSVKG RFTISRDNS ENTLYLQM NSLRAEDT AVYYCA | KAILRCIPL GCYYADAM DV (SEQ ID NO: 65) | WGQGTLVT V |
| SKA I-DS7 | EVQLLESG GGLVQTGG SLRLSCAAS GFTFS | **DYAMS**(SE Q ID NO: 41) | WVRQAPGK GLEWVS | GIYHSGGN T (SEQ ID NO: 50) | YYADSVKG RFTISRDNS KNTLYLQM NSLRAEDT AVYYCA | **RDAGPFDY** (SEQ ID NO: 66) | WGQGTLVA V |
| SKA I-DS1 0 | EVQLLESG GGLVQPGG SLRLSCAAS GFTFS | **DYAMS**(SE Q ID NO: 41) | WVRQAPGR GLEWVS | **EIYYHDSNT** (SEQ ID NO: 51) | YYADSVKG RFTISRDNS KNTLYLQM NSLRAEDT AVYYCA | **RGTGPFDY** (SEQ ID NO: 67) | WGQGTLVT V |
| SKA I-DS1 4 | EVQLLESG GGLVQPGG SLRLSCAAS GFTFS | **DYDMS(SE** Q ID NO: 43) | WVRLAPGK GLEWVS | **VISYNGGSI** (SEQ ID NO: 52) | YYADSVKG RFTISRDNS KNTLYLQM NGLRAEDT AVYYCA | KGVPEIAPP RFDY (SEQ ID NO: 68) | WGQGTLVT V |
| SKA I-DS1 5 | EVQLLESG GGLVQPGG SLRLSCAAS GFTFS | **DYDMS(SE** Q ID NO: 43) | WVRQAPGK GLEWVS | **MIYSGSSSK** (SEQ ID NO: 53) | YYADSVKG RFTISRDNS KNTLYLQM NSLRAEDT AVYYCA | RVTGTCHS GSCYYSDA MDV (SEQ ID NO: 69) | WGQGTLVT V |
| SKA I-DS1 6 | EVQLLESG GGLVQPGG SLRLSCAAS GFTFS | **DYSMS**(SE Q ID NO: 42) | WVRQAPGK GLEWVS | WIYPDNGN T (SEQ ID NO: 54) | YYADSVKG RFTISRDNS KNTLYLQM NSLRAEDT AVYYCA | **KPKTSFDY** (SEQ ID NO: 70) | WGQGTLVT V |
| SKA I-DS1 7 | EVQLLESG GGLVQPGG SLRLSCAAS GFTFS | **DYAMS**(SE Q ID NO: 41) | WVRQAPGK GLEWVS | **VIYYDGSSK** (SEQ ID NO: 55) | YYADSVKG RFTISRDNS KNTLYLQM NSLRAEDT AVYYCA | **RDFGPFDY** (SEQ ID NO: 71) | WGQGTLVT V |
| SKA I-DS4 2 | EVQLLESG GGLVQPGG SLRLSCAAS GFTFS | **NYSMS**(SE Q ID NO: 44) | WVRQAPGK GLEWVS | AISYDNGN K (SEQ ID NO: 56) | YYADSVKG RFTISRDNS KNTLYLQM NSLRAEDT AVYYCA | **RDGNTFDY** (SEQ ID NO: 72) | WGQGTLVT V |
| SKA I-DS6 3 | EVQLLESG GGLVQPGG SLRLSCAAS GFTFS | **NYAMS**(SE Q ID NO: 45) | WVRQAPGK GLEWVS | GIYPNSGSN T (SEQ ID NO: 57) | YYADSVKG RFTISRDNS KNTLYLQM NSLRAEDT | **KYQWDFDY** (SEQ ID NO: 73) | WGQGTLVT V |
| | | | | | AVYYCA | | |
| SKA I-DS6 4 | EVQLLESG GGLVQPGG SLRLSCAAS GFTFS | **DYSMS**(SE Q ID NO: 42) | WVRQAPGK GLEWVS | GIYHSDGS K (SEQ ID NO: 58) | YYADSVKG RFTISRDNS KNTLYLQM NSLRAEDT AVYYCA | **RFDPVFDY** (SEQ ID NO: 74) | WGQGTLVT V |
| SKA I-DS6 6 | EVQLLESG GGLVQPGG SLRLSCAAS GFTFS | **DYAMS**(SE Q ID NO: 41) | WVRQAPGK GLEWVS | **AIYPGGGST** (SEQ ID NO: 59) | YYADSVKG RFTISRDNS KNTLYLQM NSLRAEDT AVYYCA | **RNLFGFDY** (SEQ ID NO: 75) | WGQGTLVT V |
| SKA I-DS7 7 | EVQLLESG GGLVQTGG SLRLSCAAS GFTFS | **DYYMS**(SE Q ID NO: 46) | WVRLAPGR GLEWVS | **AISYDDGNI** (SEQ ID NO: 60) | YYADSVKG RFTISRDNS KNTLYLQM NSLRAEDT AVYYCA | **RSHPTFDY** (SEQ ID NO: 76) | WGQGTLVT V |
| SKA I-DS8 4 | EVQLLESG GGLVQPGG SLRLSCAAS GFTFS | **DYYMS**(SE Q ID NO: 46) | WVRQAPGK GLEWVS | **LISHGGSSK** (SEQ ID NO: 61) | YYADSVKG RFTISRDNS KNTLYLQM NSLRAEDT AVYYCA | RDPFPGRS SNFDY (SEQ ID NO: 77) | WGQGTLVT V |
| SKA I-DS9 5 | EVQLLESG GGLVQPGG SLRLSCAAS GFTFS | **GYDMS**(SE Q ID NO: 47) | WVRQAPGK GLEWVS | **GISYNNGST** (SEQ ID NO: 62) | YYADSVKG RFTISRDNS KNTLYLQM NSLRAEDT AVYYCA | KDAWTLHP FSFDY (SEQ ID NO: 78) | WGQGTLVT V |
| SKA I-DS1 | EVQLLESG GGLVQPGG SLRLSCAAS | **DYAMS**(SE Q ID NO: 41) | WVRQAPGK GLEWVS | WIYPDDGN T (SEQ ID NO: | YYADSVKG RFTISRDNS KNTLHLQM | **KPPTWFDY** (SEQ ID NO: 79) | WGQGTLVT V |
| 04 | GFTFS | | | 63) | NSLRAEDT AVYYCA | | |

Heavy chain variable regions of SARS-CoV2 variant virus neutralizing antibodies

### 3. Construction of SARS-CoV2 variant virus neutralizing antibodies

For conversion to human IgG neutralizing antibodies, the identified 16 scFv sequences binding to the SARS-CoV2 variant virus RBD were inserted into a human IgG two vector system (Antibody Design Labs). For the IgG conversion, the antibody-binding site was amplified using forward and reverse primers including reaction enzyme sites. The V_{H} primer was designed to have restriction sites for BspE1 at the 5'-end and Bsa1 at the 3'-end. The V_{L} primer was designed to have BssH11 and BsmB1 restriction sites at the 5'- and 3'-end, respectively. The amplified V_{H} fragment was inserted into the cloning site of a TGEC-HC vector (Antibody Design Labs), which is one of the two vector systems, and the V_{L} domain was inserted into the cloning site of a TGEX-LC vector (Antibody Design Labs). All the reaction enzymes were purchased from New England Biolabs. Plasmids were amplified using *Escherichia coli* DH5α-competent cells and all the candidate monoclonal antibodies were identified through reaction enzyme mapping and DNA sequencing (Macrogen, Korea). The cloned heavy chain and light chain IgG plasmids were transfected into ExpiCHO cells at the same time. After mixing 15 µg of each of the heavy chain and light chain human IgG plasmids with Expifectamine^{™} CHO (Thermo Fisher), the complex was co-infected into cells prepared at 6x10⁶ cells/mL. Next day, an Expifectamine^{™} CHO enhancer and an ExpiCHO^{™} medium (Thermo Fisher) were added. The cells were harvested when cell viability reached about 75% (about 7 days after the transfection). Monoclonal antibodies were purified using Protein A resin (Amicogen). Protein A agarose resin and an affinity chromatography column (Bio-Rad) were used for purification of human IgG. After mixing the produced antibodies with 1 mL of Protein A resin and incubating at 4 °C for 6 hours, the mixture was loaded in an affinity chromatography column and then eluded using 0.1 M glycine HCl buffer (pH 2.7). Finally, the purified antibodies were concentrated using 100-kDa cutoff Amicon (Merck Millipore).

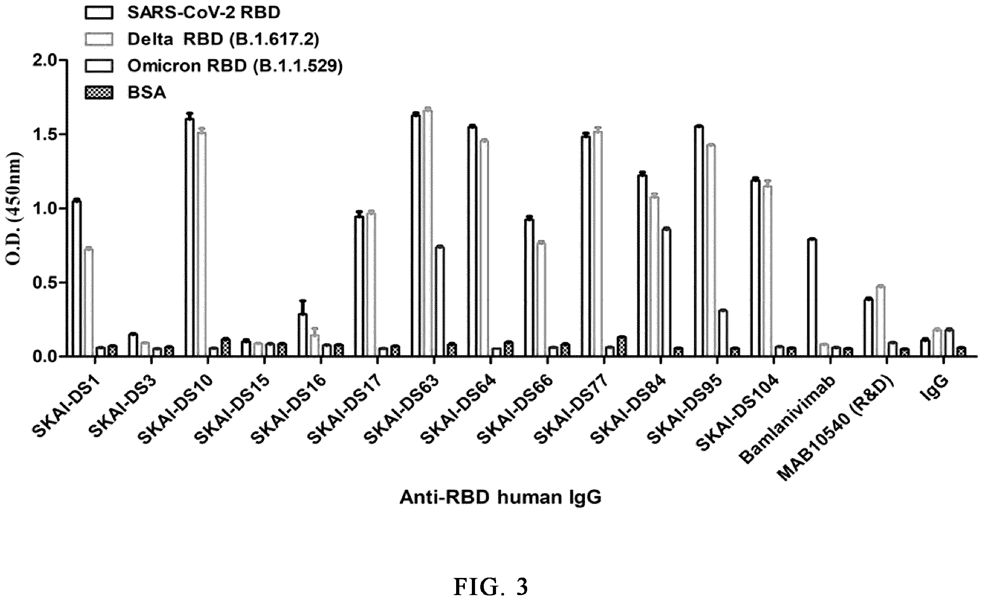

13 human IgG neutralizing antibodies were obtained from the 16 candidate scFv antibodies. The binding ability of the 13 candidate neutralizing antibodies (1 µg/mL) was investigated by ELISA using SARS-CoV2 RBD, Delta RBD and Omicron (dominant species as of January, 2022) RBD antigens (Genscript). As a result, 10 antibodies binding to SARS-CoV2 RBD, 9 antibodies binding to Delta RBD, and 3 antibodies binding to Omicron were identified. Three antibodies, i.e., SKAI-DS63, SKAI-DS84 and SKAI-DS95 were identified to bind to all the three antigens (FIG. 3). Further experiment was conducted using SKAI-DS10 that showed the best binding ability for SARS-CoV2 and Delta antigens and SKAI-DS63 and SKAI-DS84 that showed above-average binding ability for all the three antigens as test groups, bamlanivimab (research only, ProteoGenix, Eli Lilly) and MAB10540 (R&D Systems) as positive control groups, and human IgG as a negative control group.

### 4. Antibody-antigen protein thermal shift analysis (PTS)

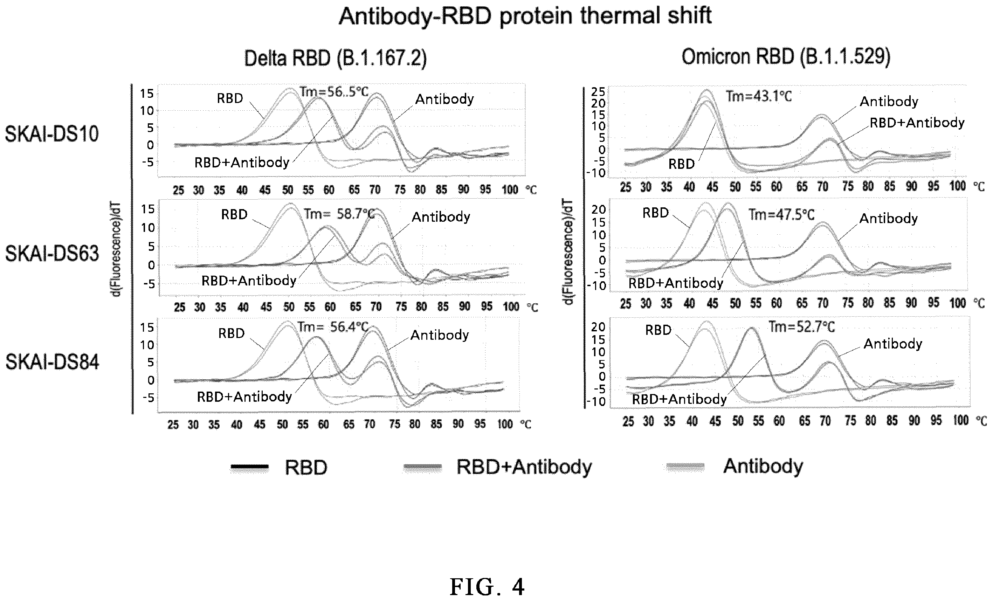

The binding ability of the screened SKAI-DS10, DS63 and DS84 antibodies was verified again through antibody-antigen protein thermal shift analysis (PTS) using Delta and Omicron RBD antigens. Protein thermal shift verifies the binding ability between an antigen and an antibody by measuring the temperate at which an antigen-antibody complex is dissociated (Tₘ) while increasing temperature with time. As a result, all the three antibodies showed good binding ability for the Delta RBD antigen, and the antigen-antibody complex was dissociated at high temperatures for Omicron RBD in the order of DS10 < DS63 < DS83. Therefore, it was confirmed that DS84 has strong binding ability to the RBD of the Omicron variant virus, which is the predominant species at present (FIG. 4).

### 5. Verification of neutralizing ability using pseudovirus system

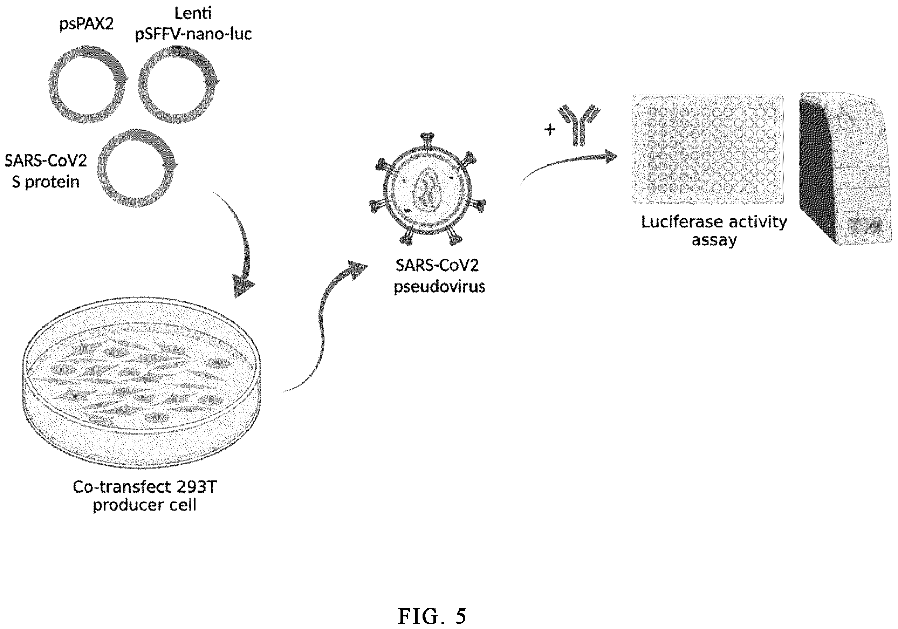

A pseudovirus system was established for verification of the neutralizing ability of the screened candidate neutralizing antibodies (FIG. 5). After co-transfecting a packaging plasmid (psPAX2), a transgene plasmid (pLenti-sffv-NanoLuc-PGK-RFP-T2A-PURO) and a plasmid expressing the SARS-CoV2 S protein into HEK293T cells, viruses were harvested 72 hours later and then concentrated 30-50 fold using a lentivirus concentrator (Takara). Then, after incubating the candidate antibody and the pseudovirus at 37 °C for 1 hour, ACE2-Huh7.5 cells were infected therewith. 24 hours later, the medium containing the virus was removed and a fresh medium containing 10% FBS was added. After incubation at 37 °C for 72 hours, the cytotoxicity of the antibody was evaluated by WST assay. In addition, nanoluciferase activity was evaluated for the same cells using Promega's NanoLuc-Glo (luciferase activity is lower as neutralizing ability is higher).

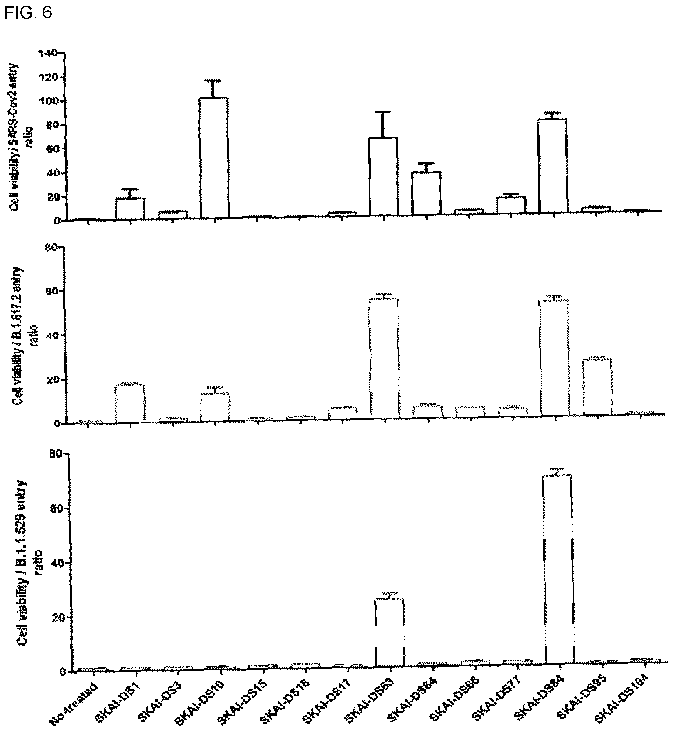

The antigen-binding ability of the 13 candidate antibodies at a concentration of 1 µg/mL was verified by ELISA (FIG. 6). In order to confirm whether they exhibit neutralizing ability consistent with the ELISA result, the neutralizing ability of the 13 candidate antibodies at a concentration of 1 µg/mL was investigated. As a result, as shown in FIG. 3, the 84th antibody showed the best neutralizing ability for Omicron variant, and the 63rd and 10th antibodies showed neutralizing ability for SARS-CoV2 and Delta variant only. Accordingly, the inventors of the present disclosure selected 10th antibody that showed the most powerful neutralizing ability for WT (Wuhan species) and Delta variant and the 64th and 84th antibodies that neutralized all of WT, Delta variant and Omicron variant as test groups. The dose-dependent neutralizing ability of the 3 antibodies was investigated using pseudoviruses.

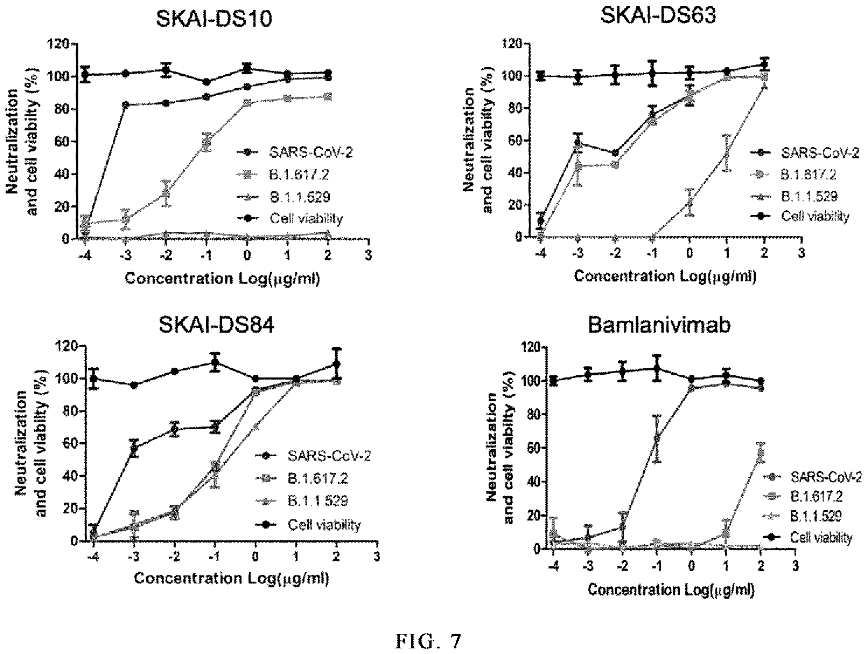

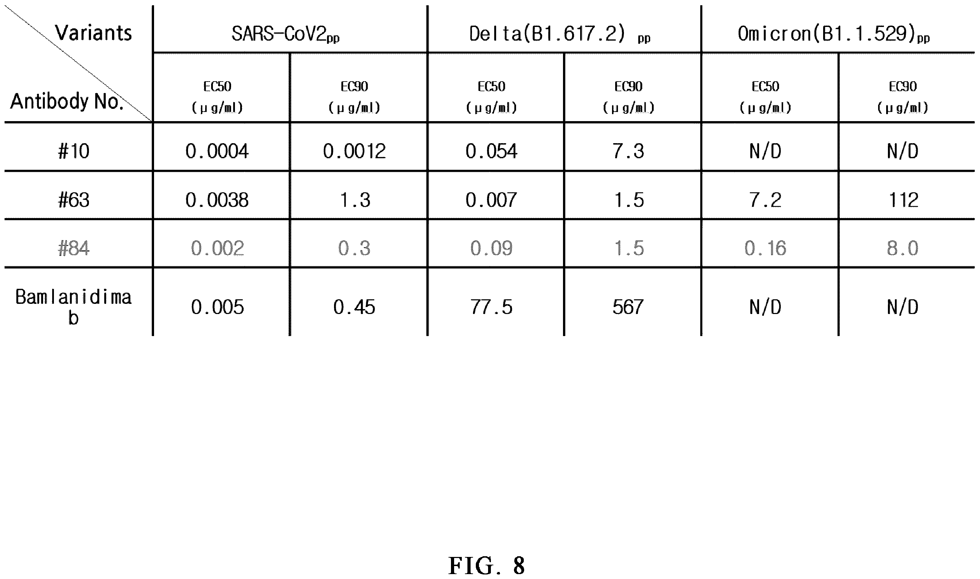

After treating the pseudoviruses of Wuhan species, Delta variant and Omicron variant with 10th, 63rd and 84th antibodies of different concentrations and conducting incubation for 72 hours, neutralizing ability was investigated by measuring nanoluciferase activity (FIG. 7). As a result, the 84th antibody showed the best neutralizing ability for Omicron variant, followed by the 63rd antibody. The 10th antibody showed no neutralizing ability for Omicron variant. The degree of inhibition of Omicron variant was EC₅₀ = 0.16 µg/mL and EC₉₀ = 8 µg/mL for the 84th antibody, and EC₅₀ = 7.2 µg/mL and EC₉₀ = 112 µg/mL for the 63rd antibody. The neutralizing ability for the Wuhan species was EC₅₀ = 0.0004 µg/mL and EC₉₀ = 0.0012-0.0015 µg/mL for the 10th and 63rd antibodies, and the neutralizing ability of the 84th antibody for the Wuhan species was lower as EC₅₀ = 0.002 µg/mL and EC₉₀ = 0.3 µg/mL. Finally, the neutralizing ability for the Delta variant was EC₅₀ = 0.003 µg/mL and EC₉₀ = 7.3 µg/mL for the 10th antibody, EC₅₀ = 0.007 µg/mL and EC₉₀ = 5.2 µg/mL for the 63th antibody, and EC₅₀ = 0.09 g/mL and EC₉₀ = 1.5 µg/mL for the 84th antibody (FIG. 8). Bamlanivimab was used as a control group. Bamlanivimab showed neutralizing ability only for the Wuhan species (FIG. 8).

### 6. Verification of neutralizing ability using neutralization test kit

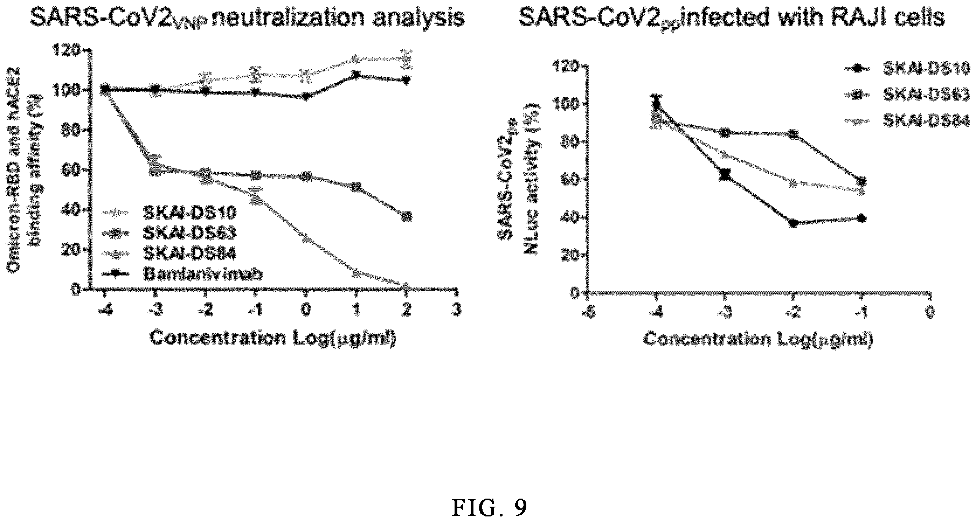

In order to re-identify the neutralizing ability verified for the pseudoviruses, neutralizing ability was evaluated again using a SARS-CoV2 surrogate virus neutralization test kit (Genscript). As a result, the 84th antibody showed the most powerful neutralizing ability, followed by the 63rd antibody, whereas the 10th antibody and bamlanivimab (control group) showed no neutralizing ability for the Omicron variant. Accordingly, the neutralizing ability of the 84th antibody for the Omicron variant was verified again (FIG. 9).

### 7. Verification of antibody-dependent-enhancement (ADE)

In order to investigate the FcγRII-dependent effect of the antibodies on antibody-dependent-enhancement (ADE), the ADE of the three antibodies was investigated using RAJI cells (ATCC, CCL-86). As a result, the infection by pseudoviruses was no observed even when the concentration of the antibodies was increased. In addition, the viral infection in the RAJI cells was decreased as the concentration of the antibodies was increased. That is to say, none of the three antibodies showed ADE effect.

### 8. Verification of antibody affinity

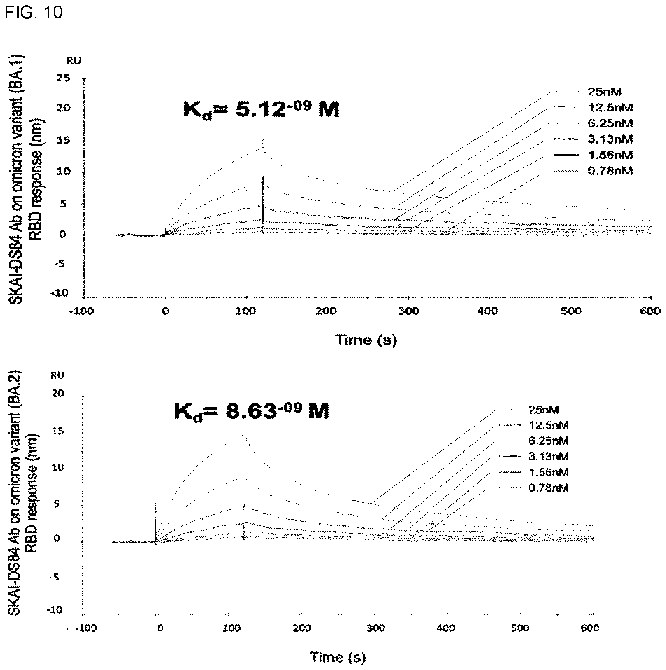

The inventors of the present disclosure selected the SKAI-DS84 antibody that showed the highest neutralizing ability for the Omicron variant as a candidate antibody and measured the binding ability (affinity) of the antibody. The antigen-antibody affinity between the SKAI-DS84 antibody and Omicron RBD BA.1 and BA.2 (Stealth Omicron) antigens purchased from InvivoGen was investigated using Biacore T200. It was confirmed that Omicron RBD BA.1 and BA.2 had four different sequences at the RBD site. It was confirmed that the affinity (K_{d}) between the SKAI-DS84 antibody and the Omicron RBD BA.1 antigen was 5.12x10⁻⁹ M, and the affinity for Omicron BA.2 was 8.63x10⁻⁹ M (FIG. 10). Accordingly, it was confirmed that the SKAI-DS84 antibody shows strong binding ability for the Omicron RBD (BA.1 and BA.2 RBD mutation sequences: S371F, T376A, D405N, R408S).

While the specific exemplary embodiments of the present disclosure have been described, those having ordinary knowledge in the art can change and modify the present disclosure within the scope of the present disclosure defined by the appended claims through addition, changes, deletion, modification, etc.

## Claims

1. A neutralizing antibody against SARS-coronavirus 2 (SARS-CoV2) or a variant virus thereof, or an antigen-binding fragment thereof, wherein
the antibody or an antigen-binding fragment thereof comprises light chain CDR1, CDR2 and CDR3 and heavy chain CDR1, CDR2 and CDR3, and
the light chain CDR1 is represented by an amino acid sequence of General Formula 1; the light chain CDR2 is represented by an amino acid sequence of General Formula 2; the light chain CDR3 is represented by an amino acid sequence of General Formula 3; the heavy chain CDR1 is represented by an amino acid sequence of General Formula 4; the heavy chain CDR2 is represented by an amino acid sequence of General Formula 5; and the heavy chain CDR3 is represented by an amino acid sequence of General Formula 6, General Formula 7 or General Formula 8:
**General Formula 1** X₁₁-G-S-S-S-N-I-G-X₁₂-X₁₃-X₁₄-V-X₁₅
wherein X₁₁ is S or T; X₁₂ is S or N; X₁₃ is N or T; X₁₄ is Y, D, A, N, T or S; and X₁₅ is S, T, Y or N;
**General Formula 2** X₂₁-X₂₂-X₂₃-X₂₄
wherein X₂₁ is S, A or D; X₂₂ is D or N; X₂₃ is N or S; and X₂₄ is N, K, H or Q;
**General Formula 3** X₃₁-X₃₂-W-D-X₃₃-S-L-X₃₄-X₃₅
wherein X₃₁ is A or G; X₃₂ is T, A or S; X₃₃ is A, Y or D; X₃₄ is S or N; and X₃₅ is A or G;
**General Formula 4** X₄₁-Y-X₄₂-M-S
wherein X₄₁ is N, D or G; and X₄₂ is A, S, Y or D;
**General Formula 5** X₅₁-I-X₅₂-X₅₃-X₅₄-X₅₅-X₅₆-X₅₇-X₅₈-X₅₉
wherein X₅₁ is W, A, G, E, V, M or L; X₅₂ is Y or S; X₅₃ is S, H, Y or P; X₅₄ is N, D, S, H or G; X₅₅ is D, S, G or N; X₅₆ is G or S; X₅₇ is N or S; X₅₈ is N, K, T or I; and X₅₉ is nonexistent or T;
**General Formula 6** X601-X602-X603-X604-X605-X606-X607-X60S-X609-X610-X611-X612-X613-X614-D-A-M-D-V
wherein X₆₀₁ is R or K; X₆₀₂ is F, V or A; X₆₀₃ is P, T or I; X₆₀₄ is V, G or L; X₆₀₅ is P, T or R; X₆₀₆ is S or C; X₆₀₇ is W, H or I; X₆₀₈ is R, S or P; X₆₀₉ is N, G or L; X₆₁₀ is T, S or G; X₆₁₁ is W or C; X₆₁₂ is S or Y; X₆₁₃ is S or Y; and X₆₁₄ is Y, S or A;
**General Formula 7** X₇₀₁ -X₇₀₂-X₇₀₃-X₇₀₄-X₇₀₅-X₇₀₆-X₇₀₇-X₇₀₈-X₇₀₉-X₇₁₀-F-D-V
wherein X₇₀₁ is R or K; X₇₀₂ is D or G; X₇₀₃ is P, A or V, X₇₀₄ is F, W or P; X₇₀₅ is P, T or E; X₇₀₆ is G, L or I; X₇₀₇ is R, H or A; X₇₀₈ is S or P; X₇₀₉ is S, For P; and X₇₁₀ is N, S or R; and
**General Formula 8** X₈₁-X₈₂-X₈₃-X₈₄-X₈₅-F-D-Y
wherein X₈₁ is R or K; X₈₂ is P, D, Y, F, N, S or G; X₈₃ is K, F, G, Q, D, L, H, P, A or T; X₈₄ is T, G, N, W, P or F; and X₈₅ is S, P, T, D, V, G or W.

2. The antibody or the antigen-binding fragment thereof according to claim 1, wherein the variant virus is Delta or Omicron variant virus.

3. The antibody or the antigen-binding fragment thereof according to claim 2, wherein the variant virus is Omicron variant virus.

4. The antibody or the antigen-binding fragment thereof according to claim 3, wherein, in the light chain CDR1 of the antibody or the antigen-binding fragment thereof, X₁₄ and X₁₅ are S or T.

5. The antibody or the antigen-binding fragment thereof according to claim 3, wherein, in the light chain CDR2 of the antibody or the antigen-binding fragment thereof, X₂₁ is A or D; X₂₂ is D; and X₂₄ is N, H or Q.

6. The antibody or the antigen-binding fragment thereof according to claim 3, wherein, in the light chain CDR3 of the antibody or the antigen-binding fragment thereof, X₃₂ is A; X₃₃ is D; and X₃₄ is S.

7. The antibody or the antigen-binding fragment thereof according to claim 3, wherein, in the heavy chain CDR1 of the antibody or the antigen-binding fragment thereof, X₄₂ is A, Y or D.

8. The antibody or the antigen-binding fragment thereof according to claim 3, wherein, in the heavy chain CDR2 of the antibody or the antigen-binding fragment thereof, X₅₁ is G or L; X₅₃ is H, Y or P; X₅₄ is N or G; X₅₅ is S, G or N; and X₅₈ is K, T or N.

9. The antibody or the antigen-binding fragment thereof according to claim 3, wherein, the heavy chain CDR3 of the antibody or an antigen-binding fragment thereof is represented by an amino acid sequence of General Formula 7 and wherein X₇₀₂ is D; X₇₀₃ is P or A, X₇₀₄ is F or W; X₇₀₅ is P or T; X₇₀₆ is G or L; X₇₀₇ is R or H; X₇₀₉ is S or F; and X₇₁₀ is N or S.

10. The antibody or the antigen-binding fragment thereof according to claim 3, wherein the heavy chain CDR3 of the antibody or an antigen-binding fragment thereof is represented by an amino acid sequence of General Formula 8 and wherein X₈₁ is K; X₈₂ is Y; X₈₃ is Q; X₈₄ is W; and X₈₅ is D.

11. The antibody or the antigen-binding fragment thereof according to claim 1, wherein the antibody or the antigen-binding fragment thereof comprises light chain CDR1, CDR2 and CDR3 and heavy chain CDR1, CDR2 and CDR3, wherein
(1) the light chain CDR1 is represented by an amino acid sequence of SEQ ID NO 9, the light chain CDR2 is represented by an amino acid sequence of SEQ ID NO 20, the light chain CDR3 is represented by an amino acid sequence of SEQ ID NO 28, the heavy chain CDR1 is represented by an amino acid sequence of SEQ ID NO 41, the heavy chain CDR2 is represented by an amino acid sequence of SEQ ID NO 48 and the heavy chain CDR3 is represented by an amino acid sequence of SEQ ID NO 64;
(2) the light chain CDR1 is represented by an amino acid sequence of SEQ ID NO 10, the light chain CDR2 is represented by an amino acid sequence of SEQ ID NO 21, the light chain CDR3 is represented by an amino acid sequence of SEQ ID NO 29, the heavy chain CDR1 is represented by an amino acid sequence of SEQ ID NO 42, the heavy chain CDR2 is represented by an amino acid sequence of SEQ ID NO 49 and the heavy chain CDR3 is represented by an amino acid sequence of SEQ ID NO 65;
(3) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 41, the heavy chain CDR2 is represented by an amino acid sequence of SEQ ID NO 50 and the heavy chain CDR3 is represented by an amino acid sequence of SEQ ID NO 66;
(4) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 41, the heavy chain CDR2 is represented by an amino acid sequence of SEQ ID NO 51 and the heavy chain CDR3 is represented by an amino acid sequence of SEQ ID NO 67;
(5) the light chain CDR1 is represented by an amino acid sequence of SEQ ID NO 12, the light chain CDR2 is represented by an amino acid sequence of SEQ ID NO 23, the light chain CDR3 is represented by an amino acid sequence of SEQ ID NO 31, the heavy chain CDR1 is represented by an amino acid sequence of SEQ ID NO 43, the heavy chain CDR2 is represented by an amino acid sequence of SEQ ID NO 52 and the heavy chain CDR3 is represented by an amino acid sequence of SEQ ID NO 68;
(6) the light chain CDR1 is represented by an amino acid sequence of SEQ ID NO 13, the light chain CDR2 is represented by an amino acid sequence of SEQ ID NO 24, the light chain CDR3 is represented by an amino acid sequence of SEQ ID NO 32, the heavy chain CDR1 is represented by an amino acid sequence of SEQ ID NO 43, the heavy chain CDR2 is represented by an amino acid sequence of SEQ ID NO 53 and the heavy chain CDR3 is represented by an amino acid sequence of SEQ ID NO 69;
(7) the light chain CDR1 is represented by an amino acid sequence of SEQ ID NO 14, the light chain CDR2 is represented by an amino acid sequence of SEQ ID NO 24, the light chain CDR3 is represented by an amino acid sequence of SEQ ID NO 33, the heavy chain CDR1 is represented by an amino acid sequence of SEQ ID NO 42, the heavy chain CDR2 is represented by an amino acid sequence of SEQ ID NO 54 and the heavy chain CDR3 is represented by an amino acid sequence of SEQ ID NO 70;
(8) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 34, the heavy chain CDR1 is represented by an amino acid sequence of SEQ ID NO 41, the heavy chain CDR2 is represented by an amino acid sequence of SEQ ID NO 55 and the heavy chain CDR3 is represented by an amino acid sequence of SEQ ID NO 71;
(9) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 35, the heavy chain CDR1 is represented by an amino acid sequence of SEQ ID NO 44, the heavy chain CDR2 is represented by an amino acid sequence of SEQ ID NO 56 and the heavy chain CDR3 is represented by an amino acid sequence of SEQ ID NO 72;
(10) the light chain CDR1 is represented by an amino acid sequence of SEQ ID NO 15, the light chain CDR2 is represented by an amino acid sequence of SEQ ID NO 25, the light chain CDR3 is represented by an amino acid sequence of SEQ ID NO 36, the heavy chain CDR1 is represented by an amino acid sequence of SEQ ID NO 45, the heavy chain CDR2 is represented by an amino acid sequence of SEQ ID NO 57 and the heavy chain CDR3 is represented by an amino acid sequence of SEQ ID NO 73;
(11) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 42, the heavy chain CDR2 is represented by an amino acid sequence of SEQ ID NO 58 and the heavy chain CDR3 is represented by an amino acid sequence of SEQ ID NO 74;
(12) the light chain CDR1 is represented by an amino acid sequence of SEQ ID NO 16, the light chain CDR2 is represented by an amino acid sequence of SEQ ID NO 24, the light chain CDR3 is represented by an amino acid sequence of SEQ ID NO 37, the heavy chain CDR1 is represented by an amino acid sequence of SEQ ID NO 41, the heavy chain CDR2 is represented by an amino acid sequence of SEQ ID NO 59 and the heavy chain CDR3 is represented by an amino acid sequence of SEQ ID NO 75;
(13) the light chain CDR1 is represented by an amino acid sequence of SEQ ID NO 11, the light chain CDR2 is represented by an amino acid sequence of SEQ ID NO 22, the light chain CDR3 is represented by an amino acid sequence of SEQ ID NO 30, the heavy chain CDR1 is represented by an amino acid sequence of SEQ ID NO 46, the heavy chain CDR2 is represented by an amino acid sequence of SEQ ID NO 60 and the heavy chain CDR3 is represented by an amino acid sequence of SEQ ID NO 76;
(14) the light chain CDR1 is represented by an amino acid sequence of SEQ ID NO 17, the light chain CDR2 is represented by an amino acid sequence of SEQ ID NO 26, the light chain CDR3 is represented by an amino acid sequence of SEQ ID NO 38, the heavy chain CDR1 is represented by an amino acid sequence of SEQ ID NO 46, the heavy chain CDR2 is represented by an amino acid sequence of SEQ ID NO 61 and the heavy chain CDR3 is represented by an amino acid sequence of SEQ ID NO 77;
(15) the light chain CDR1 is represented by an amino acid sequence of SEQ ID NO 18, the light chain CDR2 is represented by an amino acid sequence of SEQ ID NO 27, the light chain CDR3 is represented by an amino acid sequence of SEQ ID NO 39, the heavy chain CDR1 is represented by an amino acid sequence of SEQ ID NO 47, the heavy chain CDR2 is represented by an amino acid sequence of SEQ ID NO 62 and the heavy chain CDR3 is represented by an amino acid sequence of SEQ ID NO 78; or
(16) the light chain CDR1 is represented by an amino acid sequence of SEQ ID NO 19, the light chain CDR2 is represented by an amino acid sequence of SEQ ID NO 23, the light chain CDR3 is represented by an amino acid sequence of SEQ ID NO 40, the heavy chain CDR1 is represented by an amino acid sequence of SEQ ID NO 41, the heavy chain CDR2 is represented by an amino acid sequence of SEQ ID NO 63 and the heavy chain CDR3 is represented by an amino acid sequence of SEQ ID NO 79.

12. The antibody or the antigen-binding fragment thereof according to claim 1, wherein the antibody or an antigen-binding fragment thereof comprises light chain FR1, FR2, FR3 and FR4 and heavy chain FR1, FR2, FR3 and FR4, wherein the light chain FR1 is represented by an amino acid sequence of SEQ ID NO 1, the light chain FR2 is represented by an amino acid sequence of SEQ ID NO 2, the light chain FR3 is represented by an amino acid sequence of SEQ ID NO 3, the light chain FR4 is represented by an amino acid sequence of SEQ ID NO 4, the heavy chain FR1 is represented by an amino acid sequence of SEQ ID NO 5, the heavy chain FR2 is represented by an amino acid sequence of SEQ ID NO 6, the heavy chain FR3 is represented by an amino acid sequence of SEQ ID NO 7 and the heavy chain FR4 is represented by an amino acid sequence of SEQ ID NO 8.

13. A nucleic acid molecule encoding the antibody or the antigen-binding fragment thereof according to claim 1.

14. A vector comprising the nucleic acid molecule according to claim 13.

15. A cell comprising the vector according to claim 14.

16. A pharmaceutical composition for preventing or treating infection by SARS-coronavirus 2 (SARS-CoV2) or a variant virus thereof, comprising the antibody or the antigen-binding fragment thereof according to claim 1, a nucleic acid molecule encoding the antibody or an antigen-binding fragment thereof, or a vector comprising the nucleic acid molecule as an active ingredient.

17. A method for preventing or treating infection by SARS-coronavirus 2 (SARS-CoV2) or a variant virus thereof, comprising a step of administering a pharmaceutically effective amount of the antibody or the antigen-binding fragment thereof according to claim 1, a nucleic acid molecule encoding the antibody or an antigen-binding fragment thereof, or a vector comprising the nucleic acid molecule to a subject.

18. A use of the antigen-binding fragment thereof according to claim 1, a nucleic acid molecule encoding the antibody or an antigen-binding fragment thereof, or a vector comprising the nucleic acid molecule in therapy.

19. A composition for diagnosing infection by SARS-coronavirus 2 (SARS-CoV2) or a variant virus thereof, comprising the antibody or the antigen-binding fragment thereof according to claim 1, a nucleic acid molecule encoding the antibody or an antigen-binding fragment thereof, or a vector comprising the nucleic acid molecule as an active ingredient.
